(19) **Europäisches Patentamt / European Patent Office / Office européen des brevets**

(11) **EP 4 549 414 A1**

(12) **EUROPEAN PATENT APPLICATION**
published in accordance with Art. 153(4) EPC

(43) Date of publication:
07.05.2025 Bulletin 2025/19

(21) Application number: 23831646.7

(22) Date of filing: 30.06.2023

(51) International Patent Classification (IPC):
C04B 35/486 (2006.01)    A61K 6/818 (2020.01)
A61K 6/822 (2020.01)

(52) Cooperative Patent Classification (CPC):
A61K 6/818; A61K 6/822; C04B 35/486

(86) International application number:
PCT/JP2023/024507

(87) International publication number:
WO 2024/005207 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 01.07.2022 JP 2022106984

(71) Applicant: Tosoh Corporation
Yamaguchi 746-8501 (JP)

(72) Inventors:
• AZECHI Sho
Shunan-shi Yamaguchi 746-8501 (JP)
• MATSUURA Moeka
Shunan-shi Yamaguchi 746-8501 (JP)
• KAWAMURA Kiyotaka
Shunan-shi Yamaguchi 746-8501 (JP)
• NAGAYAMA Hitoshi
Shunan-shi Yamaguchi 746-8501 (JP)
• USHIO Yuki
Shunan-shi Yamaguchi 746-8501 (JP)

(74) Representative: Müller-Boré & Partner
Patentanwälte PartG mbB
Friedenheimer Brücke 21
80639 München (DE)

(54) **METHOD FOR PRODUCING SINTERED BODY**

(57) An object is to provide a method for producing a sintered body by short-time sintering, by which a sintered body satisfying the light transmitting property required of dental prostheses is obtained without requiring precision temperature control in the high-temperature range and irrespective of the stabilizing element content in the zirconia compositional substance. A method for producing a stabilizing element-containing zirconia sintered body includes a first heating step of heating a zirconia compositional substance containing a stabilizing element from a heating start temperature to a first target temperature of 800°C or higher and lower than 1400°C at a heating rate of 150°C/minute or more; a second heating step of elevating the temperature from the first target temperature to a second target temperature of 1400°C or higher and lower than 1580°C at a heating rate of more than 30°C/minute and less than 200°C/minute; and a retaining step of retaining the second target temperature.

**Description**

TECHNICAL FIELD

The present disclosure relates to methods for producing zirconia sintered bodies, and particularly relates to a method for producing a zirconia sintered body in a short time.

BACKGROUND ART

Sintered bodies containing zirconia as a main component are used in dental prosthesis applications such as crowns and bridges. A sintered body has been gaining an aesthetic merit comparable to that of natural teeth by sintering (hereinafter also referred to as "normal sintering") that includes heating from room temperature to a highest target temperature, retaining the highest target temperature, and cooling from the highest target temperature, and that requires a total of 7 hours or more including 2 hours or more for retaining the highest target temperature. Thus, a long sintering time has been necessary to produce these sintered bodies.

To address this, in recent years, studies have been conducted on a sintering method (hereinafter may also be referred to as "short-time sintering") that requires a shorter sintering time than the normal sintering and that can still produce a sintered body having an aesthetic merit comparable to that of a sintered body (hereinafter may be referred to as a "normal sintered body") obtained by normal sintering

Regarding the short-time sintering, studies have been conducted mainly on improving the heating rate to shorten the time for retaining the highest temperature and shorten the time required for sintering. For example, Patent Document 1 discloses short-time sintering that includes a three-stage heating step. Furthermore, Patent Document 2 discloses short-time sintering that includes a heating step in which the heating rate in a temperature range from 75% to 90% of the maximum temperature is increased and the heating rate thereafter is decreased so as to shorten the time for retaining the highest temperature.

PRIOR ART DOCUMENTS

PATENT DOCUMENTS

PATENT DOCUMENT 1: INTERNATIONAL PUBLICATION NO. 2021/048674
PATENT DOCUMENT 2: INTERNATIONAL PUBLICATION NO. 2019/166938

DISCLOSURE OF INVENTION

TECHNICAL PROBLEM

The short-time sintering in Patent Document 1 requires switching the heating rate in a high-temperature range exceeding 1200°C, and, in the high-temperature range exceeding 1200°C, since densification behavior caused by sintering progresses rapidly, precision temperature control is necessary. However, in switching the heating rate in the high-temperature range, precision temperature control is difficult since the heater of the sintering furnace cannot sufficiently follow the heating program; moreover, a sintering furnace that can comply with such a control is limited to a special heating furnace. Furthermore, a sintered body obtained by the short-time sintering in Patent Document 2 has a low light transmitting property compared to normal sintered bodies and lacks the light transmitting property required of the dental prostheses. Furthermore, according to the method of Patent Document 2, the light transmitting property of a stabilized zirconia sintered body having a high yttrium content is particularly low compared to normal sintered bodies.

An object of the present disclosure is to provide a method for producing a zirconia sintered body by short-time sintering, by which a sintered body having a light transmitting property required of dental prostheses can be obtained without requiring precision temperature control in the high-temperature range and irrespective of the stabilizing element content in the zirconia compositional substance.

SOLUTION TO PROBLEM

The inventors of the present invention have focused on the heating step in short-time sintering. As a result, it has been found that a sintered body that exhibits an aesthetic merit similar to that of a normal sintered body can be obtained by elevating the temperature to a highest target temperature by using a particular heating rate and a particular target temperature without necessitating precision temperature control in the high-temperature range. It has also been found that a zirconia sintered body having a light transmitting property required of dental prostheses is obtained by such a production method irrespective of the stabilizing element content in the zirconia compositional substance.

That is, the present invention is as disclosed by the claims, and the gist of the present disclosure is as follows.

[1] A method for producing a stabilizing element-containing zirconia sintered body, the method comprising: a first heating step of heating a zirconia compositional substance containing a stabilizing element from a heating start temperature to a first target temperature of 800°C or higher and lower than 1400°C at a heating rate of 150°C/minute or more; a second heating step of elevating the temperature from the first target temperature to a second target temperature of 1400°C or higher and lower than 1580°C at a heating rate of more than 30°C/minute and less than 200°C/minute; and a retaining step of retaining the second target temperature.

[2] The production method described in [1] above, wherein a retention time in the retaining step is less than 20 minutes.

[3] The production method described in [1] or [2] above, wherein a difference between the second target temperature and the first target temperature is 300°C or more and 600°C or less.

[4] The production method described in any one of [1] to [3] above, wherein the heating rate up to the second target temperature relative to the heating rate up to the first target temperature is 0.15 or more and 1.0 or less.

[5] The production method described in any one of [1] to [4 above, wherein the heating start temperature is a temperature ranging from room temperature to 500°C.

[6] The production method described in any one of [1] to [5] above, further comprising a cooling step of decreasing the temperature from the second target temperature to a cooling temperature of 800°C or higher and 1200°C or lower and discharging a sintered body from a sintering furnace.

[7] The production method described in [6] above, wherein the discharged sintered body is cooled in an air atmosphere by at least one of natural cooling and blowing of a cooling gas.

[8] The production method described in [6] or [7] above, wherein the cooling gas is at least one selected from the group consisting of air, argon, nitrogen and helium.

[9] The production method described in any one of [1] to [8] above, wherein a sintering furnace equipped with a heater containing at least one selected from the group consisting of molybdenum disilicide, silicon carbide, lanthanum chromite and carbon is used in sintering.

[10] The production method described in any one of [1] to [9] above, wherein the compositional substance is a green body or calcined body of zirconia.

[11] The production method described in any one of [1] to [10], wherein the stabilizing element is at least one selected from the group consisting of yttrium, calcium, cerium, magnesium, praseodymium, ytterbium, erbium and terbium.

[12] The production method described in any one of [1] to [11] above, wherein a content of the stabilizing element is 2.5 mol% or more and 8 mol% or less.

[13] The production method described in any one of [1] to [12] above, wherein the compositional substance has a T + C phase ratio of 65% or more.

[14] The production method according to any one of Claims 1 to 13, wherein the compositional substance contains at least one coloring element selected from transition metal elements other than zirconium and hafnium and lanthanoid rare earth elements.

ADVANTAGEOUS EFFECTS OF INVENTION

The present disclosure achieves the object of providing a method for producing a sintered body by short-time sintering, by which a sintered body satisfying a light transmitting property required of dental prostheses is obtained without requiring precision temperature control in the high-temperature range and irrespective of the stabilizing element content in the zirconia compositional substance.

DESCRIPTION OF EMBODIMENTS

A method for producing a sintered body according to the present disclosure will now be described through one example embodiment. The terms used in the embodiments are as follows. Each of the features and parameters disclosed in this description may be combined in any desired manner, and upper limits and lower limits of values disclosed in this description may be combined in any desired manner.

A "compositional substance" is a substance having a particular composition, and is, for example, at least one selected from the group consisting of a powder, a green body, a calcined body and a sintered body. A "zirconia compositional substance" is a compositional substance containing zirconia as a main component, in particular, a compositional substance essentially consisting of zirconia.

A "powder" is a set of powder particles (particles that are primary particles, secondary particles or both) and is a compositional substance having flowability. A "zirconia powder" is a powder containing zirconia as a main component, in particular, a powder essentially consisting of zirconia. Furthermore, a "powder compositional substance" is a compositional substance constituted by powders having different features, in particular, a compositional substance containing powders having different compositions.

A "granular powder" is a set of aggregates (granular particles) of powder particles and is a compositional substance having flowability, in particular, a compositional substance in a state where the powder particles have slowly aggregated. A "zirconia granular powder" is a granular powder containing zirconia as a main component, in particular, a granular powder essentially consisting of zirconia.

A "green body" is a compositional substance that has a particular shape constituted by powder particles aggregated by a physical force, in particular, a compositional substance in a state where heat treatment is not yet performed after the shape is given (for example, after forming). A "zirconia green body" is a green body containing zirconia as a main component, in particular, a green body essentially consisting of zirconia. Here, the green body is interchangeable with a "compact".

A "calcined body" is a compositional substance that has a particular shape constituted by fused particles, in particular, a compositional substance heat-treated at a temperature lower than a sintering temperature. A "zirconia calcined body" is a calcined body containing zirconia as a main component, in particular, a calcined body essentially consisting of zirconia.

A "sintered body" is a compositional substance that has a particular shape constituted by crystal grains, in particular, a compositional substance heat-treated at a temperature higher than or equal to the sintering temperature. A "zirconia sintered body" is a sintered body containing zirconia as a main component, in particular, a sintered body essentially consisting of zirconia.

A "main component" is a component that forms a main phase (matrix, base material, or base phase) in the composition of the compositional substance, and the mass ratio of the main component relative to the compositional substance is preferably 75 mass% or more, 85 mass% or more, 90 mass% or more, 95 mass% or more, 98 mass% or more or 99 mass% or more and 100 mass% or less or less than 100 mass%.

A "stabilizing element" is an element that stabilizes zirconia crystal phases by dissolving into zirconia.

The stabilizing element content in the compositional substance (mol%, hereinafter, this content may be referred to as "stabilizing element amount") is a molar ratio of the stabilizing element as oxide relative to the total of zirconium as $ZrO_2$ and the stabilizing element as oxide in the compositional substance.

A "BET specific surface area" is a specific surface area $[m^2/g]$ measured in accordance with JIS R 1626 by a BET multipoint method (5 points) using nitrogen as adsorption gas, in particular, a BET specific surface area measured under the following conditions.

Adsorption medium: $N_2$
Adsorption temperature: -196°C
Pretreatment conditions: air atmosphere, degassing treatment at 250°C for 1 hour or longer

The BET specific surface area can be measured by using a generally available specific surface area meter (for example, TriStar II 3020 produced by Shimadzu Corporation). The calcined body is processed into a 5 mm × 5 mm × 16 mm cuboid shape, and all of the surfaces of this cuboid are polished with a #400 grit sandpaper in accordance with JIS R 6001-2 to prepare a measurement sample.

An "average particle size" is $D_{50}$ in a volume-based particle size distribution of a powder measured by a wet method, and can be measured with a generally available apparatus (for example, MT3300EXII produced by MicrotracBEL Corp.). A powder may be subjected to a dispersing treatment such as an ultrasonic treatment to remove slow aggregation and then dispersed in pure water to form a slurry, and this slurry may be used as a measurement sample. The volume-based particle size distribution measurement by a wet method is preferably performed by adjusting the pH of the slurry to 3 to 6.

An "average granule size" is $D_{50}$ in a volume-based particle size distribution of a granular powder measured by a dry method, and can be measured with a generally available apparatus (for example, MT3100II produced by MicrotracBEL Corp.). The measurement sample may be a granular powder in a slow aggregation state without being subjected to a dispersing treatment such as an ultrasonic treatment.

A "powder X-ray diffraction pattern" is an XRD pattern obtained by performing smoothing and background subtraction on an XRD pattern, which is obtained by performing a powder X-ray diffraction (hereinafter may also be referred to as "XRD") measurement on the compositional substance under the following conditions, by using an analyzing program that comes with an X-ray diffractometer (for example, Integrated Analytical Software for Powder X-ray Diffraction, PDXL Ver. 2.2 produced by RIGAKU Corporation).

Radiation source: CuKα radiation ($\lambda$ = 0.15418 nm)

Measurement mode: continuous scan
Scan speed: 2°/minute
Measurement range:

$2\theta$ = 26° to 33°
$2\theta$ = 72° to 76°

Accelerating voltage/current:40 mA/40 kV
Divergence height slit: 10 mm
Divergence/incident slit: 1°
Receiving slit: open
Detector: semiconductor detector (D/teX Ultra)
Filter: Ni filter
Goniometer radius: 185 mm

XRD measurement may be carried out by using a common X-ray diffractometer (for example, Ultima IV produced by RIGAKU Corporation). The calcined body may have surfaces polished with a #400 grit sandpaper in accordance with JIS R 6001-2 and then lap-polished by using a 3 $\mu$m grit diamond abrasive to prepare a measurement sample, and the XRD measurement may be performed on the lap-polished surface.

An "XRD peak" is a peak that has a peak top at $2\theta$ detected in an XRD pattern obtained by the aforementioned XRD measurement. In this embodiment, "having no XRD peak" means that the XRD peak is not detected in the aforementioned XRD measurement.

Examples of the XRD peaks corresponding to the respective crystal planes of zirconia are those XRD peaks that have peak tops at $2\theta$ below:

XRD peak corresponding to monoclinic (111) plane: $2\theta$ = 31 $\pm$ 0.5°
XRD peak corresponding to monoclinic (11-1) plane: $2\theta$ = 28 $\pm$ 0.5°
XRD peak corresponding to tetragonal (111) plane: $2\theta$ = 30 $\pm$ 0.5°
XRD peak corresponding to cubic (111) plane: $2\theta$ = 30 $\pm$ 0.5°

The XRD peak corresponding to the tetragonal (111) plane and the XRD peak corresponding to the cubic (111) plane are measured as one overlapping peak.

A "T + C phase ratio" is the ratio of the tetragonal and cubic phases in the zirconia crystal phases, is a ratio of the area intensity of the XRD peak of the tetragonal and cubic zirconia relative to the total area intensity of the XRD peaks of the tetragonal, cubic, and monoclinic zirconia in the XRD pattern obtained in the aforementioned XRD measurement, and is determined from the following equation.

$$f_{T+C} = [I_t(111) + I_c(111)] / [I_m(111) + I_m(11-1) + I_t(111) + I_c(111)]$$

In the equation above, $f_{T+C}$ represents the tetragonal and cubic phase ratio, $I_t(111)$ represents the area intensity of the tetragonal (111) plane, $I_c(111)$ represents the area intensity of the cubic (111) plane, $I_m(111)$ represents the area intensity of the monoclinic (111) plane, $I_m(11-1)$ represents the area intensity of the monoclinic (11-1) plane, and $I_t(111) + I_c(111)$ corresponds to the area intensity of the XRD peak that has a peak top at $2\theta$ = 30 $\pm$ 0.5°.

The area intensity of each XRD peak is a value obtained by analyzing the XRD pattern by using an analyzing program that comes with an X-ray diffractometer (for example, Integrated Analytical Software for Powder X-ray Diffraction, PDXL Ver. 2.2 produced by RIGAKU Corporation).

The "green body density" is an actually measured density [g/cm$^3$] of a green body, and is a mass [g] of the green body obtained by measuring the mass with a balance relative to the volume [cm$^3$] of the green body obtained from dimensions measured with a caliper.

The "calcined body density" is an actually measured density [g/cm$^3$] of a calcined body, and is a mass [g] of the calcined body obtained by measuring the mass with a balance relative to the volume [cm$^3$] of the calcined body obtained from dimensions measured with a caliper.

A "Vickers hardness" is a value measured by using a common Vickers hardness tester (for example, Q30A produced by Qness GmbH) equipped with a square-based pyramid diamond indenter. In the measurement, the indenter is statically pressed into a measurement sample surface, and the diagonal length of the indentation mark formed in the measurement sample surface is measured. The obtained diagonal length is used to determine the Vickers hardness from the equation below.

$$Hv = F/\{d^2/2\sin(\alpha/2)\}$$

In the equation above, Hv represents the Vickers hardness (HV), F represents a measurement load (1 kgf), d represents the diagonal length (mm) of the indentation mark, and $\alpha$ represents the face angle (136°) of the indenter.

Examples of the measurement conditions for the Vickers hardness are as follows.

Measurement sample: disk shape with a thickness of 3.0 $\pm$ 0.5 mm
Measurement load: 1 kgf

Prior to the measurement, the measurement sample may be subjected to a pretreatment by polishing the measurement surface with a #800 water-resistant abrasive paper to remove irregularities exceeding 0.1 mm.

A "total transmittance" is the ratio [%] of transmitted light (total of regular transmitted light and diffuse transmitted light) relative to incident light measured in accordance with JIS K 7361-1 from a measurement sample having a sample thickness of 1.0 $\pm$ 0.1 mm. A disk-shaped sintered body having a sample thickness of 1.0 $\pm$ 0.1 mm and a surface roughness Ra $\leq$ 0.02 $\mu$m in both surfaces may be used as the measurement sample, and a haze meter (for example, haze meter NDH4000 produced by NIPPON DENSHOKU INDUSTRIES Co., Ltd.) equipped with a D65 light source as the light source may be used as the measurement instrument.

A "biaxial flexural strength" is a value determined by a two-point bending test according to JIS T 6526. The biaxial flexural strength is measured by using a circular sintered body having a diameter of 14.5 mm $\pm$ 0.5 and a thickness of 1.25 mm $\pm$ 0.05 mm as a measurement sample, and the average value taken from 10 times of measurement with a supporting circle radius of 6 mm and an indenter radius of 0.7 mm may be used as the biaxial flexural strength of the sintered body.

"Pressureless sintering" is a method for sintering a material to be sintered (such as a green body or calcined body) by heating the material at a temperature (hereinafter may also be referred to as a "sintering temperature") at which sintering of zirconia proceeds without applying external force to the material.

"Pressureless firing" is a method for heating a subject to be treated without adding external force during a heat treatment, in particular, a method for performing heating at a temperature lower than the sintering temperature without applying external force to the subject to be treated during the heat treatment in the calcining step.

[Method for producing sintered body]

A production method according to this embodiment is a method for producing a stabilizing element-containing zirconia sintered body, the method comprising: a first heating step of heating a zirconia compositional substance containing a stabilizing element from a heating start temperature to a first target temperature of 800°C or higher and lower than 1400°C at a heating rate of 150°C/minute or more; a second heating step of elevating the temperature from the first target temperature to a second target temperature of 1400°C or higher and lower than 1580°C at a heating rate of more than 30°C/minute and less than 200°C/minute; and a retaining step of retaining the second target temperature. According to such a production method, a sintered body having a light transmitting property comparable to that of a normal sintered body can be obtained substantially without being affected by the stabilizing element amount even when the time the second target temperature is retained is shortened compared to the retention time required for normal sintering. In particular, this effect is particularly prominent for zirconia having a large stabilizing element amount. One of the reasons why the light transmitting property of the sintered body obtained by the production method of this embodiment is little affected by the stabilizing element amount is the implementation of the first heating step and the second heating step, as a result of which the influence of the difference in grain growth rate during sintering derived by the difference in stabilizing element amount is suppressed. It is considered that, as a result, pore elimination inside the zirconia compositional substance tends to be accelerated, and, furthermore, even with a compositional substance having a large stabilizing element amount in which the grain growth rate during sintering is high, pore elimination inside the compositional substance is accelerated.

In the first heating step of this embodiment, a stabilizing element-containing zirconia compositional substance is heated from a heating start temperature to a first target temperature of 800°C or higher and lower than 1400°C at a heating rate of 150°C/minute or more (hereinafter this first target temperature is also referred to as "T1", and the heating rate from the heating start temperature to T1 is also referred to as "HR1"). When HR1 is less than this, it takes an excessively long time to reach the temperature at which sintering prominently progresses. In addition, when HR1 satisfies this rate, the compositional substance is evenly heated, and the temperature unevenness between the inside and the surface of the compositional substance is decreased. As a result, progress of sintering in the second heating step described below and pore elimination resulting therefrom are smoothly accelerated. Furthermore, the time taken for heating can be shortened. Thus, a sintered body having a light transmitting property comparable to that of a normal sintered body can be obtained despite a shorter retention time compared to the highest target temperature retention time in the normal sintering and irrespective of the stabilizing element amount. The first heating step may be performed by placing the compositional substance in a sintering furnace.

The heating start temperature may be any temperature before the onset of the sintering-induced shrinkage of the compositional substance, is a temperature in the range of room temperature ($20 \pm 20°C$) to 500°C and is preferably in the range of room temperature to 100°C. To simplify temperature control and reduce the temperature unevenness between the surface and inside of the compositional substance at the start of the second heating step, the heating start temperature of the first heating step is preferably room temperature.

From the viewpoint of further shortening the time taken for the first heating step, HR1 is 180°C/minute or more, 200°C/minute or more or 230°C/minute or more, and the upper limit of HR1 is 500°C/minute or less, 400°C/minute or less, 300°C/minute or less or 280°C/minute or less, for example. HR1 is preferably 180°C/minute or more and 500°C/minute or less, 200°C/minute or more and 400°C/minute or less, 230°C/minute or more and 300°C/minute or less or 230°C/minute or more and 280°C/minute or less. Preferably, the temperature is elevated from the heating start temperature to T1 without fluctuation of the heating rate (HR1) ($\pm$ 2°C/min, preferably $\pm$1°C/min).

T1 is a temperature of 800°C or higher and lower than 1400°C. T1 is preferably a temperature lower than 1400°C, 1300°C or lower, 1200°C or lower, 1170°C or lower, 1150°C or lower, 1100°C or lower or 1050°C or lower. When T1 is 1400°C or higher and when the stabilizing element amount is large, a sintered body having a low light transmitting property compared to a normal sintered body is obtained.

When T1 is 850°C or higher, preferably 950°C or higher or 1000°C or higher, a sintered body can be produced without requiring an excessively long time for sintering. Preferable examples of T1 include 850°C or higher and 1300°C or lower, 950°C or higher and 1200°C or lower and 1000°C or higher and 1050°C or lower. When T1 is within this range, coarse pores are inhibited from becoming trapped in the compositional substance, and pores can be efficiently eliminated during the second heating step.

T1 is the switching temperature from HR1 to HR2, and thus there is no need to retain T1. However, as long as the effects of the production method of this embodiment are not impaired, the temperature may be retained at T1. When T1 is to be retained, examples thereof include 0 minute or more and 5 minutes or less and 0 minute or more and 15 seconds or less.

In the second heating step of this embodiment, the temperature is elevated from T1 to a second target temperature of 1400°C or higher and lower than 1580°C at a heating rate of more than 30°C/minute and less than 200°C/minute (hereinafter, the second target temperature may be referred to as "T2" and the heating rate from T1 to T2 may be referred to as "HR2"). From a temperature exceeding T1 to T2 in the second heating step, switching of the heating rate is not necessary. Since precision temperature control (controlling the heating rate) in the high-temperature range is not necessary, the production method of this embodiment can be adopted to a production method that uses a sintering furnace equipped with a general-purpose heater. In a temperature range of 1400°C or higher, sintering of zirconia progresses rapidly, and the sintering behavior varies depending on the composition and the physical properties of the compositional substance, etc. Thus, it is difficult to switch the heating rate in the temperature range of 1400°C or higher according to the composition and properties of the compositional substance. According to the production method of the present embodiment, there is no switching of the heating rate during heating from 1400°C or higher to T2, nor is there switching of the heating rate during heating from T1 to T2. In this manner, the heating rate does not change after exceeding 1200°C before reaching T2, and thus a sintered body having a high light transmitting property can be obtained with a simple heating program.

In the second heating step, pores are gradually eliminated from the inside of the composite, and this creates a form in which pores are mainly present near the surface. For this, HR2 is, for example, 150°C/minute or less, 100°C/minute or less, 80°C/minute or less or 60°C/minute or less. Meanwhile, from the viewpoint of shortening the sintering time, HR2 is to be 33°C/minute or more, 35°C/minute or more or 40°C/minute or more, and is preferably 33°C/minute or more and 150°C/minute or less or 35°C/minute or more and 80°C/minute or less. At HR2 within these ranges, the higher the heating rate, the higher the light transmitting property of the resulting sintered body from a compositional substance having a small stabilizing element amount. In contrast, the lower the heating rate, the higher the light transmitting property of the resulting sintered body from a compositional substance having a stabilizing element amount. To easily obtain a sintered body having a high light transmitting property irrespective of the stabilizing element amount, HR2 is preferably 40°C/minute or more and 180°C/minute or less and further preferably 45°C/minute or more and 120°C or less. Preferably, the temperature is elevated from T1 to T2 without fluctuation of the heating rate (HR2) ($\pm$ 2°C/min, preferably $\pm$1°C/min).

HR1 and HR2 are preferably HR1 > HR2, and HR2 relative to HR1 (hereinafter may also be referred to as "HR2/HR1") is preferably 0.15 or more and 1.0 or less and more preferably 0.2 or more and 0.5 or less.

T2 is the highest target temperature in the production method of this embodiment and is 1400°C or higher and lower than 1580°C. When T2 is within this range, pore elimination by grain growth of zirconia crystal grain proceeds. As a result, a sintered body that satisfies a light transmitting property required as the dental prostheses is obtained by short-time sintering irrespective of the stabilizing element amount. In addition, since the highest target temperature is lower than 1580°C, deterioration of a sintering furnace equipped with a general-purpose heater, such as a molybdenum silicide ($MoSi_2$) heater or silicon carbide (SiC) heater, is reduced. For example, deterioration of silicon carbide occurs at 1580°C or higher. Thus, when T2 is within this range, deterioration of a silicon carbide heater is reduced and a sintered body having a light transmitting property comparable to that of a normal sintered body can be produced.

In order to easily obtain a sintered body that satisfies a mechanical strength required of the dental prostheses, T2 is 1450°C or higher, 1480°C or higher or 1500°C or higher, and 1570°C or lower, 1565°C or lower, 1560°C or lower or lower than 1560°C, for example. In the production method of this embodiment, pores in the compositional substance are preferably present near the surface before reaching T2. From this viewpoint, T2 is preferably 1500°C or higher and more preferably 1510°C or higher. In this manner, a sintered body having a light transmitting property comparable to that of a normal sintered body is obtained even when the T2 retention time is short such as 15 minutes or less or even 10 minutes or less. T2 is preferably 1500°C or higher and 1565°C or lower or 1500°C or higher and lower than 1560°C.

The difference between T2 and T1 which are T2 > T1 is, for example, 150°C or more and less than 780°C, 200°C or more and less than 780°C, 300°C or more and 600°C or less or 500°C or more and 600°C or less.

It is considered that, in this manner, the first heating step and the second heating step are not merely steps aimed at elevating the temperature to T2 but also serve as pore elimination steps that alter the structure of a body to be sintered into a structure in which pore elimination is accelerated.

In addition, the production method of this embodiment does not require knowing, prior to sintering, the details of the sintering-induced shrinkage behavior of the compositional substance such as the temperature before reaching the particular density and sintering rates, and a compositional substance can be sintered by controlling the temperature program for sintering. Thus, the method is suitable as a method for producing a wide variety of sintered bodies such as dental prostheses for which the individual differences are very large for each patient.

The production method of this embodiment has a retaining step of retaining T2. The production method of this embodiment is a sintering method that has two heating steps: first heating step and second heating step. It is considered that this feature moves the pores inside the compositional substance to the surface and creates a state where pores are present near the surface at the time T2 is reached. Thus, even when the T2 retention time is short, the pores near the surface of the compositional substance are eliminated, and a sintered body having a light transmitting property comparable to the normal sintered body is obtained.

The retention time in the retaining step may be 1 minute or more, 3 minutes or more or 5 minutes or more. In order to shorten the time required for the production method of this embodiment and cut down the energy consumption required for the production method of this embodiment, the retention time is preferably short. The upper limit of the retention time is, for example, 30 minute or less, 20 minute or less, less than 20 minutes or 15 minute or less. The retention time at the highest target temperature is preferably short, and since T2 is 1500°C or higher, in particular, 1510°C or higher, the light transmitting property of a sintered body obtained rarely decreases despite the short T2 retention time. Thus, the T2 retention time is preferably 1 minute or more and less than 20 minutes. When the retention time is within these ranges, the shorter the retention time, the higher the light transmitting property of the resulting sintered body for a compositional substance having a small stabilizing element amount. In contrast, the longer the retention time, the higher the light transmitting property of the resulting sintered body for a compositional substance having a large stabilizing element amount. To more easily obtain a sintered body having a high light transmitting property irrespective of the stabilizing element amount, the retention time is preferably 1 minute or more and 15 minutes or less and more preferably 5 minutes or more and 15 minutes or less.

In the production method of this embodiment, a sintered body is obtained by performing the retaining step. The sintered body after the retaining step may be cooled by any method with which a desired sintered body is obtained, and be recovered from the sintering furnace. The production method of this embodiment preferably has a cooling step of decreasing the temperature from T2 to a cooling temperature (hereinafter also referred to as "T3") of 800°C or higher and 1200°C or lower, more preferably has a cooling step of decreasing the temperature from T2 to T3 and discharging the sintered body from the sintering furnace, and yet more preferably has a cooling step of decreasing the temperature from T2 to T3, discharging the sintered body from the sintering furnace and cooling the discharged sintered body in an air atmosphere by at least one of natural cooling and blowing of a cooling gas.

To further shorten the cooling time, the cooling rate (hereinafter also referred to as "CR1") from T2 to T3 is 30°C/minute or more, 40°C/minute or more, 45°C/minute or more or 50°C/minute or more. In addition, CR1 is, for example, 200°C/minute or less, 150°C/minute or less, 100°C/minute or less or 80°C/minute or less, and, in this manner, defects such as cracks are rarely generated during cooling. CR1 is preferably 30°C/minute or more and 200°C/minute or less or 45°C/minute or more and 80°C/minute or less, and the cooling rate (CR1) from T2 to T3 is preferably free of fluctuation ($\pm 2$°C/min preferably $\pm 1$°C/min).

The sintered body may be discharged from the sintering furnace after being cooled to T3. By cooling to T3, generation of defects due to heat shock is reduced even when the sintered body is discharged from the sintering furnace and exposed to an air atmosphere at room temperature, and the change in color tone of a coloring element-containing sintered body during cooling is also reduced. T3 is, for example, 800°C or higher or 850°C or higher, and 1200°C or lower, 1100°C or lower, 1050°C or lower or 950°C or lower, and is preferably 800°C or higher and 1100°C or lower. To further suppress changes in color tone of the obtained sintered body, T3 is more preferably 800°C or higher and lower than 1000°C and yet more preferably 850°C or higher and 950°C or lower.

The discharged sintered body is further cooled. The sintered body may be cooled to a temperature such that the sintered body can be handled (for example, 100°C or lower or room temperature). In order to decrease nonuniformity in strength of the obtained sintered bodies between different sintering lots, the sintered body discharged from the sintering furnace is more preferably cooled from T3 to 400°C at an average cooling rate (hereinafter also referred to as "$CR2_{(AVE)}$") of 300°C/minute or more, 350°C/minute or more or 400°C/minute or more. In addition, for example, $CR2_{(AVE)}$ is 800°C/minute or less, 700°C/minute or less, 600°C/minute or less or 500°C/minute or less. More preferably, cooling is performed at $CR2_{(AVE)}$ of 300°C/minute or more and 800°C/minute or less, 350°C/minute or more and 600°C/minute or less or 400°C/minute or more and 600°C/minute or less. Cooling from T3 to 400°C does not use the sintering program of the sintering furnace, and thus the cooling rate may fluctuate.

$CR2_{(AVE)}$ may be determined by bringing a common thermocouple (for example, testo735-1 produced by Testo SE & Co. KGaA) into contact with the surface of a sintered body to measure the time taken for the temperature to reach 400°C from T3 and determining $CR2_{(AVE)}$ from {(T3 - 400) [°C]/time taken for temperature to reach 400°C from T3 [minute]}.

To obtain such $CR2_{(AVE)}$, the sintered body discharged from the sintering furnace may be naturally cooled but is preferably cooled by blowing a cooling gas. The cooling gas is, for example, at least one selected from the group consisting of air, argon (Ar), nitrogen ($N_2$) and helium (He), in particular, air.

The sintered body cooled to 400°C may be cooled to a desired temperature by a desired method such as at least one of natural cooling and blowing of a cooling gas.

Examples of the time taken for the cooling step is 30 seconds or more, 1 minute or more, 2 minutes or more or 3 minutes or more, and 10 minutes or less, 8 minutes or less or 5 minutes or less, and the time is preferably 30 second or more and 10 minutes or less, 1 minute or more and 8 minutes or less or 3 minutes or more and 5 minutes or less.

As described above, the method for producing a zirconia sintered body includes a first heating step of heating a zirconia compositional substance containing a stabilizing element from a heating start temperature to T1 at a heating rate of 150°C/minute or more; a second heating step of elevating the temperature from T1 to T2 at a heating rate of more than 30°C/minute and less than 200°C/minute; and a retaining step of retaining T2, and a sintered body is obtained by sintering the compositional substance by using a sintering furnace.

Here, the first heating step, the second heating step, the retaining step and temperature control in cooling from T2 to T3 may be carried out by a sintering program of a sintering furnace used in sintering.

The sintering method that can be applied in the production method of this embodiment is, for example, at least one selected from the group consisting of vacuum sintering, pressure sintering and pressureless sintering, or at least one of pressure sintering and pressureless sintering. However, the sintering method in the production method of this embodiment is preferably pressureless sintering and is more preferably only pressureless sintering.

The atmosphere of the production method of this embodiment, that is, the atmosphere during the first heating step, the second heating step, the retaining step and the cooling step is preferably an oxidizing atmosphere and is more preferably an air atmosphere.

The sintering furnace that can be used in the production method of this embodiment may be any, for example, at least one selected from the group consisting of a resistance heating furnace, an induction heating furnace, a high-frequency furnace and an IH furnace, or at least one selected from the group consisting of a resistance heating furnace, an induction heating furnace and an IH furnace. The production method of this embodiment is more preferably a production method that performs sintering by using at least one of a resistance heating furnace and an induction heating furnace.

The sintering furnace used in sintering is preferably a sintering furnace equipped with a general-purpose heater (heating body), a sintering furnace equipped with a heater that contains at least one selected from the group consisting of molybdenum disilicide ($MoSi_2$), silicon carbide (SiC), lanthanum chromite ($LaCrO_3$) and carbon (C) or a sintering furnace equipped with a heater that contains at least one of molybdenum disilicide and silicon carbide. The production method of this embodiment is preferably a production method that performs sintering by using a sintering furnace equipped with a heater containing at least one selected from the group consisting of molybdenum disilicide, silicon carbide, lanthanum chromite and carbon and is more preferably a production method that performs sintering by using a sintering furnace equipped with a silicon carbide heater since these sintering furnaces are generally used as sintering furnaces for dental prostheses.

Examples of the time taken for the production method of this embodiment (in particular, from the first heating step to the rapid cooling step) is 15 minutes or more, 20 minutes or more, 25 minutes or more, 30 minutes or more or 35 minutes or more, and 55 minutes or less, 50 minutes or less, 45 minutes or less or 40 minutes or less, and the time is preferably 15 minutes or more and 55 minutes or less, 25 minutes or more and 50 minutes or less, 30 minutes or more and 45 minutes or less or 35 minutes or more and 40 minutes or less.

The production method of this embodiment is suitable as a method for producing a zirconium sintered body, in particular, a method for producing a zirconia sintered body for dental prostheses. In addition, the production method of this embodiment is suitable as a method for producing an yttrium-containing zirconia sintered body, in particular, a method for producing an yttrium-containing zirconia sintered body having an yttrium content of 2.8 mol% or more and 6 mol% or less, a method for producing an yttrium-containing zirconia sintered body having an yttrium content of 4 mol% or more and 6 mol% or less or a method for producing an yttrium-containing zirconia sintered body having an yttrium content of 4.5 mol% or more and 6 mol% or less.

Furthermore, this embodiment can be deemed as a program for sintering a stabilizing element-containing zirconia compositional substance, the program including: first heating involving elevating the temperature from a sintering start temperature to a first target temperature of 800°C or higher and lower than 1400°C at a heating rate of 150°C/minute or more; second heating of elevating the temperature from the first target temperature to a second target temperature of 1400°C or higher and lower than 1580°C at a heating rate of more than 30°C/minute and less than 200°C/minute; and retaining the second target temperature.

Furthermore, this embodiment can be deemed as a program for sintering a stabilizing element-containing zirconia compositional substance, which is a sintering method that includes first heating involving elevating the temperature from a sintering start temperature to a first target temperature of 800°C or higher and lower than 1400°C at a heating rate of 150°C/minute or more; second heating of elevating the temperature from the first target temperature to a second target temperature of 1400°C or higher and lower than 1580°C at a heating rate of more than 30°C/minute and less than 200°C/minute; and retaining the second target temperature.

[Compositional substance]

The compositional substance used in the production method of this embodiment is a compositional substance containing zirconia as a main component.

Zirconia may contain inevitable impurities such as hafnia ($HfO_2$). The amount of hafnia contained as an inevitable impurity varies depending on the raw material ore or the production method, and, for example, is 2.0 mass% or less. In calculating the values based on the composition, such as the composition and density, in this embodiment, hafnia may be deemed as zirconia ($ZrO_2$).

Zirconia preferably contains a stabilizing element and is more preferably zirconia containing a dissolved stabilizing element (hereinafter also referred to as "stabilizing element-dissolved zirconia"). The stabilizing element preferably contains at least yttrium, for example, at least one selected from the group consisting of yttrium, calcium, cerium, magnesium, praseodymium, ytterbium, erbium and terbium, preferably contains at least one selected from the group consisting of yttrium, cerium, erbium and terbium, more preferably contains at least one selected from the group consisting of yttrium, erbium and terbium and yet more preferably is yttrium.

The stabilizing element amount in the compositional substance may be any amount that stabilizes the zirconia crystal phases, and may be, for example, 0.1 mol% or more and 10 mol% or less. When the stabilizing element is yttrium, the stabilizing element amount (when the stabilizing element is yttrium or the like, this is referred to as "yttrium amount" or the like) is, for example, 2.5 mol% or more, 3 mol% or more, 3.5 mol% or more, 4 mol% or more or 4.5 mol% or more, and 8 mol% or less, 7 mol% or less, 6 mol% or less or 5.5 mol% or less, and is preferably 2.5 mol% or more and 8 mol% or less, 3 mol% or more or 6 mol% or less, 3.5 mol% or more and 6 mol% or less or 4.5 mol% or more and 5.5 mol% or less. According to the method of this embodiment, a sintered body having a light transmitting property comparable to that of a normal sintered body can be obtained even when a compositional substance having a large stabilizing element amount, such as a compositional substance having a stabilizing element amount of 4.5 mol% or more and 6 mol% or less or even 5.1 mol% or more and 5.8 mol% or less, is sintered.

The compositional substance of this embodiment is preferably free of undissolved stabilizing elements, in particular, free of oxides of stabilizing elements, and is more preferably free of undissolved yttria ($Y_2O_3$). Whether or not undissolved stabilizing elements are contained in the compositional substance can be determined by the XRD pattern thereof. In other words, the absence of any XRD peaks of oxides of stabilizing elements (for example, $Y_2O_3$) in the XRD pattern of the compositional substance may be deemed as the absence of undissolved stabilizing elements.

The compositional substance may contain at least one selected from the group consisting of aluminum, germanium, silicon and lanthanum, at least one selected from the group consisting of aluminum, germanium and silicon (hereinafter may also be referred to as additive elements), at least one of aluminum and germanium, or aluminum. Here, the compositional substance may be free of additive elements.

The additive element content (hereinafter may also be referred to as the "additive element amount", and when the additive element is aluminum or the like, the additive element amount is also referred to as the "aluminum amount" or the like) is 0 mass% or more, more than 0 mass%, 0.02 mass% or more or 0.05 mass% or more and less than 0.2 mass%, 0.15 mass% or less, 0.1 mass% or less or 0.07 mass% or less, for example, and is preferably more than 0 mass% and 0.2 mass% or less or 0.05 mass% or more and 0.07 mass% or less.

The form of the additive element in the compositional substance may be any. An example of the form is oxide, for example, aluminum is contained as alumina ($Al_2O_3$), germanium is contained as germania ($Ge_2O_3$) and silicon is contained as silica ($SiO_2$). In addition, for example, lanthanum is contained in the form of at least one of a solid solution in zirconia and an oxide ($La_2O_3$).

The compositional substance may contain an element that has a function of coloring zirconia (hereinafter may also be referred to as a "coloring element"). The coloring element is, for example, at least one of transition metal elements and lanthanoid rare earth elements, in particular, at least one of transition metal elements other than zirconium (Zr) and hafnium (Hf) and lanthanoid rare earth elements. Examples of the preferable coloring element include at least one selected from the group consisting of iron (Fe), cobalt (Co), manganese (Mn), nickel (Ni), copper (Cu), titanium (Ti), chromium (Cr), praseodymium (Pr), neodymium (Nd), erbium (Er), terbium (Tb) and ytterbium (Yb), at least one selected from the group consisting of iron, cobalt, manganese, nickel, copper, titanium, chromium and neodymium, at least one selected from the group consisting of iron, cobalt, titanium, manganese, praseodymium, erbium and terbium, at least one selected from the group consisting of iron, cobalt, titanium, erbium and terbium, or at least one selected from the group consisting of iron, cobalt, titanium and erbium, and the coloring element preferably contains at least iron or terbium and more preferably contains at least iron. Here, praseodymium, ytterbium, erbium and terbium each function as a coloring element and a stabilizing element.

Particularly preferable examples of the coloring element include at least two selected from the group consisting of iron, cobalt, titanium and erbium, and iron and at least one selected from the group consisting of cobalt, titanium and erbium.

The type and content of the coloring element may be any depending on the color tone of the desired sintered body. Depending on the type of the coloring element, the content thereof (hereinafter may also be referred to as the "coloring element amount", and, when the coloring element or the like is iron, the content thereof is referred to as the "iron amount" or the like) varies; however, each coloring element content is, for example, 0 mass% or more, more than 0 mass%, 0.002 mass% or more or 0.02 mass% or more and 0.5 mass% or less, 0.2 mass% or less or 0.1 mass% or less, and is preferably 0 mass% or more and 0.5 mass% or less, more than 0 mass% and 0.5 mass% or less or 0.002 mass% or more and 0.2 mass% or less.

The total amount of the coloring elements is, for example, 0 mass% or more and 0.5 mass% or less, more than 0 mass% and 0.5 mass% or less, 0.05 mass% or more and 0.5 mass% or less or 0.1 mass% or more and 0.45 mass% or less.

The form of the coloring element contained in the compositional substance may be any. For example, the coloring element may be contained as at least one of an ion and a compound. Alternatively, the coloring element may be dissolved in zirconia to be contained.

The compositional substance may contain a binder. A known binder used in forming ceramics can be used as the binder, and an organic binder is preferable. The organic binder is, for example, at least one selected from the group consisting of polyvinyl alcohol, polyvinyl butyrate, wax and acrylic resin, and is preferably at least one of polyvinyl alcohol and acrylic resin and more preferably acrylic resin. In this embodiment, the acrylic resin is a polymer that contains at least one of acrylic acid ester and methacrylic acid ester. Specific examples of the acrylic resin include at least one selected from the group consisting of polyacrylic acid, polymethacrylic acid, acrylic acid copolymers and methacrylic acid copolymers, and derivatives thereof. A specific example of the binder is at least one selected from the group consisting of AS-1100, AS-1800 and AS-2000 (all trade names, produced by Toa Gosei Co., Ltd.).

The stabilizing element amount in the compositional substance may be determined as a molar ratio [mol%] of the stabilizing element as oxide relative to the total of zirconium and the stabilizing element as oxide, the additive element amount may be determined as a mass ratio [mass%] of the additive element as oxide relative to the compositional substance as oxide and the coloring element amount may be determined as a mass ratio [mass%] of the additive element as oxide relative to the compositional substance as oxide. For example, the composition of a compositional substance that contains zirconia as a main component and that contains yttrium and erbium as the stabilizing element, aluminum as the additive element and iron and titanium as the coloring element is determined as follows.

$$\text{Mass of compositional substance}$$

$$= (ZrO_2 + Y_2O_3 + Al_2O_3 + Er_2O_3 + Fe_2O_3 + TiO_2) \ [g]$$

Stabilizing element amount (total)

$$\{(Er_2O_3 + Y_2O_3) \ [mol]/(ZrO_2 + Y_2O_3 + Er_2O_3) \ [mol]\} \times 100$$

Yttrium amount

$$\{Y_2O_3 \ [mol]/(ZrO_2 + Y_2O_3 + Er_2O_3) \ [mol]\} \times 100$$

Erbium amount (as stabilizing element)

$$\{(Er_2O_3) \, [mol]/(ZrO_2 + Y_2O_3 + Er_2O_3) \, [mol]\} \times 100$$

Additive element amount (aluminum amount)

$$\{Al_2O_3 \, [g]/(ZrO_2 + Y_2O_3 + Al_2O_3 + Er_2O_3 + Fe_2O_3 + TiO_2) \, [g]\} \times 100$$

Coloring element amount

$$\{(Er_2O_3 + Fe_2O_3 + TiO_2) \, [g]/(ZrO_2 + Y_2O_3 + Al_2O_3 + Er_2O_3 + Fe_2O_3 + TiO_2) \, [g]\} \times 100$$

Iron amount

$$\{Fe_2O_3 \, [g]/(ZrO_2 + Y_2O_3 + Al_2O_3 + Er_2O_3 + Fe_2O_3 + TiO_2) \, [g]\} \times 100$$

Titanium amount

$$\{TiO_2 \, [g]/(ZrO_2 + Y_2O_3 + Al_2O_3 + Er_2O_3 + Fe_2O_3 + TiO_2) \, [g]\} \times 100$$

Erbium amount (as coloring element)

$$\{Er_2O_3 \, [g]/(ZrO_2 + Y_2O_3 + Al_2O_3 + Er_2O_3 + Fe_2O_3 + TiO_2) \, [g]\} \times 100$$

The oxide conversion of each element is, for example, $ZrO_2$ for zirconium, $Y_2O_3$ for yttrium, CaO for calcium, $CeO_2$ for cerium, MgO for magnesium, $Pr_6O_{11}$ for praseodymium, $Yb_2O_3$ for ytterbium, $Er_2O_3$ for erbium, $Tb_7O_{11}$ for terbium, $Al_2O_3$ for aluminum, $Ge_2O_3$ for germanium, $SiO_2$ for silicon, $La_2O_3$ for lanthanum, $Fe_2O_3$ for iron, $Co_3O_4$ for cobalt, $MnO_2$ for manganese, NiO for nickel, CuO for copper, $TiO_2$ for titanium, $Cr_2O_3$ for chromium and $Nd_2O_3$ for neodymium

The compositional substance may be any compositional substance that can serve as a precursor of the sintered body, and is, for example, at least one selected from the group consisting of a powder, a granular powder, a green body and a calcined body of zirconia, in particular, at least one of a green body and a calcined body of zirconia; however, a green body or a calcined body is preferable and a calcined body is more preferable.

Here, when the compositional substance is a calcined body, the production method of this embodiment may include a step of calcining a green body of this embodiment (hereinafter, also referred to as the "calcining step") before the first heating step.

Calcining may be any heat treatment under conditions where zirconia powder particles turn into fused particles, for example, a heat treatment at a temperature lower than the sintering temperature of zirconia. Examples of the calcining conditions are as follows.

Calcining atmosphere: oxidizing atmosphere, preferably air atmosphere
Calcining temperature: 800°C or higher, 900°C or higher or 950°C or higher and lower than 1200°C, 1150°C or lower or 1100°C or lower
Calcining time: 0.5 hours or more or 1 hour or more and 5 hours or less or 3 hours or less

Here, the calcining atmosphere may be the same or different from the atmosphere for sintering.

When the compositional substance is a powder, zirconia contained in the compositional substance is preferably zirconia in a state in which a zirconia sol is heat-treated, is more preferably zirconia in a state where a zirconia sol obtained by hydrolysis of a zirconium compound is heat-treated and is yet more preferably zirconia in a state where a zirconia sol obtained by hydrolysis of zirconium oxychloride is heat-treated.

The lower limit of the BET specific surface area of the powder is, for example, 5 $m^2/g$ or more, 6 $m^2/g$ or more, 8 $m^2/g$ or more, 9 $m^2/g$ or more or 10 $m^2/g$ or more, the upper limit is, for example, 15 $m^2/g$ or less, 13 $m^2/g$ or less or 11 $m^2/g$ or less, and the BET specific surface area is preferably 5 $m^2/g$ or more and 15 $m^2/g$ or less.

The average particle size of the powder is, for example, 0.35 $\mu$m or more or 0.40 $\mu$m or more and 0.55 $\mu$m or less or 0.50 $\mu$m or less, and is preferably 0.35 $\mu$m or more and 0.55 $\mu$m or less.

Regarding the crystal phase of the powder, at least one of cubic phase and tetragonal phase preferably constitutes the main phase, and more preferably cubic and tetragonal phases constitute the main phase; and the T + C phase ratio is, for example, 65% or more, 70% or more or 75% or more, the T + C phase ratio is, for example, 100% or less, 99% or less, 98% or less, 95% or less or 93% or less, and the T + C phase ratio is preferably 65% or more and 100% or less or 75% or more and 93% or less.

The powder may be a granular powder to increase the flowability. The average granule size of the granular powder is 30 $\mu$m or more, 40 $\mu$m or more or 50 $\mu$m or more and 80 $\mu$m or less or 60 $\mu$m or less, for example, and is preferably 30 $\mu$m or more and 80 $\mu$m or less or 50 $\mu$m or more and 60 $\mu$m or less.

The untamped density of the granular powder is 1.0 g/cm$^3$ or more or 1.1 g/cm$^3$ or more and 1.4 g/cm$^3$ or less or 1.3 g/cm$^3$ or less and is preferably 1.0 g/cm$^3$ or more and 1.4 g/cm$^3$ or less or 1.1 g/cm$^3$ or more and 1.3 g/cm$^3$ or less.

When the compositional substance is a green body, the shape of the green body is, for example, at least one selected from the group consisting of a cubic shape, a cuboid shape, a polygonal shape, a columnar shape, a cylindrical shape, a disk shape and a substantially spherical shape, and may be the same shape as the sintered body to be obtained by taking into account the sintering-induced shrinkage.

The green body density is, for example, 2.8 g/cm$^3$ or more, 2.9 g/cm$^3$ or more or 3.0 g/cm$^3$ or more and 3.5 g/cm$^3$ or less, 3.4 g/cm$^3$ or less, 3.3 g/cm$^3$ or less, 3.2 g/cm$^3$ or less or 3.1 g/cm$^3$ or less, and is preferably 2.8 g/cm$^3$ or more and 3.5 g/cm$^3$ or less or 3.0 g/cm$^3$ or more and 3.1 g/cm$^3$ or less.

When the compositional substance is a calcined body, the shape of the calcined body is, for example, at least one selected from the group consisting of a cubic shape, a cuboid shape, a polygonal shape, a columnar shape, a cylindrical shape, a disk shape and a substantially spherical shape, and may be the same shape as the sintered body to be obtained by taking into account the sintering-induced shrinkage.

Regarding the crystal phase of the calcined body, at least one of cubic phase and tetragonal phase constitutes the main phase, and preferably cubic and tetragonal phases constitute the main phase; and the T + C phase ratio is, for example, 75% or more, 80% or more, 90% or more, 95% or more or 98% or more and 100% or less or 99% or less, and is preferably 75% or more and 100% or less or 98% or more and 100% or less.

The BET specific surface area of the calcined body is, for example, 5 m$^2$/g or more or 6 m$^2$/g or more and 11 m$^2$/g or less, 10 m$^2$/g or less or 9 m$^2$/g or less, and is preferably 5 m$^2$/g or more and 11 m$^2$/g or less, 6 m$^2$/g or more and 10 m$^2$/g or less or 7 m$^2$/g or more and 9 m$^2$/g or less.

The calcined body density is, for example, 2.9 g/cm$^3$ or more, 3.0 g/cm$^3$ or more or 3.1 g/cm$^3$ or more and 3.5 g/cm$^3$ or less, 3.3 g/cm$^3$ or less, or 3.2 g/cm$^3$ or less, and is preferably 2.9 g/cm$^3$ or more and 3.5 g/cm$^3$ or less, 3.0 g/cm$^3$ or more and 3.3 g/cm$^3$ or less or 3.1 g/cm$^3$ or more and 3.2 g/cm$^3$ or less.

The Vickers hardness of the calcined body of this embodiment is, for example, 35 kgf/mm$^2$ or more or 40 kgf/mm$^2$ or more and 100 kgf/mm$^2$ or less, 80 kgf/mm$^2$ or less, 70 kgf/mm$^2$ or less, 55 kgf/mm$^2$ or less or 50 kgf/mm$^2$ or less since defects caused by shape processing, such as CAM processing, are reduced.

Prior to sintering, the calcined body may be processed into a desired shape by a processing method such as CAD/CAM processing. As such, the calcined body used in the production method of this embodiment may be a calcined body in a processed state.

[Method for producing compositional substance]

The compositional substance used in the production method of this embodiment may be at least one selected from the group consisting of a powder, a granular powder, a green body and a calcined body obtained by a known production method.

When the compositional substance is a powder, a powder of zirconia or stabilizing element-containing zirconia may be obtained by at least one selected from the group consisting of a coprecipitation method, a hydrolysis method and a sol-gelt method, is more preferably obtained by at least one of a neutralization coprecipitation method and a hydrolysis method and is yet more preferably obtained by a hydrolysis method. If necessary, additive elements and the like may be mixed with the obtained powder to prepare a compositional substance used in the production method of this embodiment.

The compositional substance may contain two or more stabilizing element-dissolved zirconias having different stabilizing element amounts, and may be a powder compositional substance that contains two or more stabilizing element-dissolved zirconias having different stabilizing element amounts. A powder compositional substance that contains two or more stabilizing element-dissolved zirconias having different stabilizing element amounts is obtained by mixing two or more stabilizing element-dissolved zirconia powders having different stabilizing element amounts. The mixing method may be any desired method that can prepare a powder having a homogenous composition, and examples thereof include at least one of dry mixing and wet mixing, in particular, wet mixing or mixing in a water solvent, and mixing of slurries containing a stabilizing element-dissolved zirconia powder.

When the compositional substance is a green body, the method for producing the green body may be any, and a known ceramic forming method may be used to form the powder compositional substance. The forming method is, for example, at least one selected from the group consisting of uniaxial pressing, cold isostatic pressing, slip casting and injection molding, is preferably a forming method other than slip casting, is more preferably at least one of uniaxial pressing and cold isostatic pressing, and is yet more preferably uniaxial pressing followed by cold isostatic pressing. For example, the pressure for uniaxial pressing is 15 MPa or more and 150 MPa or less, the pressure for cold isostatic pressing is 90 MPa or more and 400 MPa or less, and the higher the pressure in forming, the higher the green body density before reaching the equilibrium.

When the green body contains a binder, a step of removing the binder, a.k.a., a debinding step, may be performed before calcining. The method for removing the binder may be any, and an example thereof is a heat treatment in an air atmosphere at 400°C or higher and lower than 800°C.

When the compositional substance is a calcined body, the calcined body may be obtained by calcining the green body as in the calcining step described above.

[Zirconia sintered body]

A sintered body obtained by the production method of this embodiment (hereinafter, also referred to as the "sintered body of this embodiment") will now be described.

The composition of the sintered body of this embodiment may be the same as the compositional substance and the calcined body described above.

The total transmittance of the sintered body of this embodiment is, for example, 25% or more, 30% or more or 35% or more and 55% or less, 53% or less or 51% or less, and is preferably 25% or more and 55% or less, 30% or more and 53% or less or 35% or more and 51% or less.

The sintered body of this embodiment preferably has a light transmitting property that can impart the same visibility as a normal sintered body obtainable from the precursor similar to the precursor of the sintered body. An example of such a light transmitting property is that the ratio of the total transmittance of the sintered body of this embodiment relative to the total transmittance of a normal sintered body (hereinafter, this ratio may be referred to as the "transmittance ratio") is 0.95 or more or 0.96 or more and 1.05 or less or 1.00 or less, or 0.95 or more and 1.05 or less or 0.96 or more and 1.00 or less.

An example of the normal sintered body referred in this embodiment is a sintered body obtained by sintering in a sintering pattern that involves elevating the temperature from room temperature to 1500°C at a heating rate of 10°C/minute in an air atmosphere, retaining 1500°C for 120 minutes and then decreasing the temperature from 1500°C to room temperature at a cooling rate of 10°C/minute.

For example, the biaxial flexural strength of the sintered body of this embodiment is 800 MPa or more and 1400 MPa or less. The sintered body of this embodiment becomes adoptable to a connected bridge for 4 or more teeth based on JIS T 6526 when the biaxial flexural strength is 800 MPa or more. The biaxial flexural strength is, for example, 830 MPa or more or 900 MPa or more and 1300 MPa or less, 1200 MPa or less or 1100 MPa or less and is preferably 830 MPa or more and 1300 MPa or less or 900 MPa or more and 1100 MPa or less.

The shape of the sintered body of this embodiment is, for example, at least one selected from the group consisting of a cubic shape, a cuboid shape, a polygonal shape, a columnar shape, a cylindrical shape, a disk shape and a substantially spherical shape, and is preferably a shape that can be used as a dental material, in particular, dental prostheses.

The sintered body of this embodiment is preferably a sintered body that can be used as a dental material, in particular, dental prosthesis material.

EXAMPLES

Hereinafter, this embodiment is described in detail through examples. However, this embodiment is not limited to these examples.

(Compositional analysis)

The composition of the compositional substance was measured by ICP analysis.

(BET specific surface area)

The BET specific surface area was determined by a BET multipoint method (5 points) by using an automatic specific surface area analyzer (TriStar II 3020 produced by Shimadzu Corporation) in accordance with JIS R 1626 under the following conditions:

Adsorption medium: $N_2$
Adsorption temperature: -196°C

Pretreatment conditions: air atmosphere, degassing treatment at 250°C for 1 hour or longer

When the calcined body was the measurement sample, prior to the measurement, the calcined body was processed into a 5 mm × 5 mm × 16 mm cuboid shape, and all of the surfaces of this cuboid were polished with a #400 grit sandpaper in accordance with JIS R 6001-2.

(Average granule size)

The average granule size was measured by loading a granular powder sample into a microtrac particle size distribution meter (apparatus name: MT3100II produced by MicrotracBEL Corp.). The particle size at 50% cumulative volume was assumed to be the average granule size.

(Crystal phase and T + C phase ratio)

The crystal phases were identified by using an X-ray diffractometer (apparatus name: Ultima IV produced by RIGAKU Corporation) by XRD measurement under the following conditions.

Radiation source: CuK$\alpha$ radiation ($\lambda$ = 0.15418 nm)
Measurement mode: continuous scan
Scan speed: 2°/minute
Measurement range:

$2\theta$ = 26° to 33°
$2\theta$ = 72° to 76°

Accelerating voltage/current: 40 mA/40 kV
Divergence height slit: 10 mm
Divergence/incident slit: 1°
Receiving slit: open
Detector: semiconductor detector (D/teX Ultra)
Filter: Ni filter
Goniometer radius: 185 mm

Before the measurement, the surfaces of the calcined body were polished with a #400 grit sandpaper in accordance with JIS R 6001-2 and then lap-polished by using a 3 $\mu$m grit diamond abrasive.
Identification of the crystal phases and calculation of the area intensity of each crystal plane were done by smoothing and background subtraction using an analysis program (program name: Integrated Analytical Software for Powder X-ray Diffraction, PDXL Ver. 2.2 produced by RIGAKU Corporation) that came with the X-ray diffractometer and then profile-fitting the processed XRD pattern by using a split pseudo-Voigt function.
The T + C phase ratio was determined from the equation described above from the XRD pattern of the calcined body of this embodiment.

(Average cooling rate)

In the rapid cooling step, a thermocouple (apparatus name: testo735-1 produced by Testo SE & Co. KGaA) was brought into contact with a surface of a sintered body to measure the time taken for the temperature to reach 400°C from the cooling temperature and the value determined from {(cooling temperature - 400) [°C]/time taken for temperature to reach 400°C from cooling temperature [minute]} was assumed to be the average cooling rate.

(Calcined body density)

The mass of the calcined body sample was measured with a balance, and the volume was determined from the dimensions measured with a caliper. The calcined body density was determined from the obtained mass and volume.

(Total transmittance)

The total transmittance of the sample was measured by a method in compliance with JIS K 7361. A standard light source D65 was used to irradiate the measurement sample, and the light flux that passed through the measurement sample was detected with an integrating sphere to measure the total transmittance. For the measurement, a haze meter (apparatus name: haze meter NDH4000 produced by NIPPON DENSHOKU INDUSTRIES Co., Ltd.) was used.

A disk-shaped sintered body 20 mm in diameter and 1.0 ± 0.1 mm in sample thickness and mirror-polished until the surface roughness Ra of both sides was 0.02 $\mu$m or less was used as the measurement sample.

(Biaxial flexural strength)

The biaxial flexural strength was measured by a method in compliance with JIS T 6526. The measurement was performed 10 times and the average value thereof was determined. The measurement was performed on a circular sintered body sample having a diameter of 14.5 mm ± 0.5 and a thickness of 1.25 mm ± 0.05 mm, with a supporting circle radius of 6 mm and an indenter radius of 0.7 mm. The crosshead speed was 0.5 mm/minute.

Synthesis example 1

A mold having an inner diameter of 25 mm was filled with 3 g of a commercially available zirconia powder (product name: Zpex produced by TOSOH CORPORATION), and uniaxial press-forming was performed at a pressure of 49 MPa. After forming, a CIP process was performed at a pressure of 196 MPa to obtain a disk-shaped green body having a diameter of 25 mm.

The obtained green body was heated at a heating rate of 50°C/hour up to 1000°C and retained at 1000°C for 1 hour to obtain a calcined body composed of alumina-containing yttrium-stabilized zirconia having an yttrium content of 3.0 mol% and an aluminum amount of 0.05 mass% and used as the calcined body of this synthesis example. The BET specific surface area of the calcined body of Synthesis example 1 was 9.1 $m^2$/g.

Synthesis example 2

A calcined body composed of yttrium-stabilized zirconia that contained alumina and the balance having an yttrium amount of 4.0 mol% and that had an aluminum amount of 0.05 mass% was obtained as in Synthesis example 1 except that a commercially available zirconia powder (product name: Zpex4 produced by TOSOH CORPORATION) was used, and used as the calcined body of this synthesis example. The BET specific surface area of the calcined body of this synthesis example was 7.4 $m^2$/g.

Synthesis example 3

Commercially available zirconia powders (Zpex4, Zpex4-Yellow and Zpex-Gray all produced by TOSOH CORPORATION) were mixed at Zpex4:Zpex4-Yellow:Zpex-Gray = 30:60:10 in terms of mass ratio to obtain a powder mixture. A calcined body composed of yttrium-stabilized zirconia that contained alumina, iron oxide and cobalt oxide and had an yttrium amount of 4.0 mol%, an aluminum amount of 0.05 mass%, an iron amount of 0.095 mass% and a cobalt amount of 0.0045 mass% was obtained as in Synthesis example 1 except that the resulting powder mixture was used, and used as the calcined body of this synthesis example. The BET specific surface area of the calcined body of this synthesis example was 7.5 $m^2$/g.

Synthesis example 4

A mixed slurry was obtained by mixing a slurry of an yttrium-stabilized zirconia powder (BET specific surface area: 14.1 $m^2$/g) having an yttrium amount of 2.5 mol% (hereinafter may also be referred to as the "2.5Y slurry") and a slurry of an yttrium-stabilized zirconia powder (BET specific surface area: 10.9 $m^2$/g) having an yttrium amount of 5.5 mol% (hereinafter may also be referred to as the "5.5Y slurry") so that the yttrium amount was 5.2 mol%. The mixed slurry was spray-dried at 180°C to obtain a granular powder of yttrium-stabilized zirconia having an yttrium amount of 5.2 mol%. The BET specific surface area of the obtained granular powder was 11.2 $m^2$/g and the average granule size was 46 $\mu$m.

A calcined body composed of yttrium-stabilized zirconia having an yttrium amount of 5.2 mol% was obtained as in Synthesis example 1 except that the obtained granular powder was used, and used as the calcined body of this synthesis example.

Synthesis example 5

The 2.5Y slurry, the 5.5Y slurry, a slurry containing 10 mass% of an iron oxide ($Fe_2O_3$) powder and a slurry containing 45 mass% of an erbium-stabilized zirconia powder having an erbium amount of 11.7 mass% (4.1 mol%) were mixed so that the yttrium amount was 5.0 mol%, the iron amount was 0.072 mass% and the erbium amount was 0.34 mass% (0.12 mol%) to obtain a mixed slurry. A granular powder that contained iron oxide, erbium-stabilized zirconia and yttrium-stabilized zirconia and had an yttrium amount of 5.0 mol%, an iron amount of 0.072 mass% and an erbium amount of 0.34 mass% (0.12 mol%) was obtained as in Synthesis example 4 except that the obtained mixed slurry was used. The BET specific surface area of the obtained granular powder was 11.4 $m^2/g$ and the average granule size was 47 $\mu m$.

A calcined body containing iron that contained iron oxide, erbium-stabilized zirconia and yttrium-stabilized zirconia and had an yttrium amount of 5.0 mol%, an iron amount of 0.072 mass% and an erbium amount of 0.34 mass% (0.12 mol%) was obtained as in Synthesis example 1 except that the obtained granular powder was used, and used as the calcined body of this synthesis example.

Synthesis example 6

The 2.5Y slurry, the 5.5Y slurry, a slurry containing 10 mass% of an iron oxide powder, a slurry containing 10 mass% of a cobalt oxide ($Co_3O_4$) powder and a slurry containing 10 mass% of titanium oxide ($TiO_2$) were mixed so that the yttrium amount was 5.2 mol%, the iron amount was 0.105 mass%, the cobalt amount was 0.0063 mass% and the titania amount was 0.0314 mass% to obtain a mixed slurry. A granular powder of yttrium-stabilized zirconia that contained iron oxide, cobalt oxide and titanium oxide and had an yttrium amount of 5.2 mol%, an iron amount of 0.105 mass%, a cobalt amount of 0.0063 mass% and a titanium amount of 0.0314 mass% was obtained as in Synthesis example 4 except that the obtained mixed slurry was used. The BET specific surface area of the obtained granular powder was 11.0 $m^2/g$ and the average granule size was 48 $\mu m$.

A calcined body of yttrium-stabilized zirconia that contained iron oxide, cobalt oxide and titanium oxide and had an yttrium amount of 5.2 mol%, an iron amount of 0.105 mass%, a cobalt amount of 0.0063 mass% and a titanium amount of 0.0314 mass% was obtained as in Synthesis example 1 except that the obtained mixed granular powder was used, and used as the calcined body of this synthesis example.

The evaluation results of the calcined bodies obtained in these synthesis examples are indicated in the table below.

[Table 1]

| | Composition | | | | | | T+C phase ratio | Calcined body density [g/cm³] |
|---|---|---|---|---|---|---|---|---|
| | $Y_2O_3$ [mol%] | $Al_2O_3$ [wt%] | $Fe_2O_3$ [wt%] | $Co_3O_4$ [wt%] | $Er_2O_3$ [wt%] | $TiO_2$ [wt%] | | |
| Synthesis example 1 | 3.0 | 0.05 | - | - | - | - | 100% | 3.21 |
| Synthesis example 2 | 4.0 | 0.05 | - | - | - | - | 100% | 3.23 |
| Synthesis example 3 | 4.0 | 0.05 | 0.095 | 0.0045 | - | - | 100% | 3.22 |
| Synthesis example 4 | 5.2 | 0 | - | - | - | - | 100% | 3.11 |
| Synthesis example 5 | 5.0 | 0 | 0.072 | - | 0.34 | - | 100% | 3.13 |
| Synthesis example 6 | 5.2 | 0 | 0.105 | 0.0063 | - | 0.0314 | 100% | 3.13 |

<Sintering pattern>

By using a sintering furnace equipped with a silicon carbide heater, the temperature was elevated in an air atmosphere from room temperature according to the sintering pattern indicated in the table below so as to perform pressureless sintering on the calcined bodies obtained in the synthesis examples. After cooling to T3, the sintered bodies after sintering were discharged from the sintering furnace and cooled to 400°C by blowing air to the sintered bodies in an air atmosphere.

[Table 2]

| Sintering pattern | First heating step | | Second heating step | | Retaining step | Cooling | | | |
|---|---|---|---|---|---|---|---|---|---|
| | HR1 (°C/minute) | T1 (°C) | HR2 (°C/minute) | T2 (°C) | Retention time (minute) | T3 (°C) | CR1 (°C/minute) | Time from T2 to T3 (minute) | CR2(AVE) (°C/minute) |
| P1 | 250 | 1000 | 50 | 1550 | 10 | 900 | 50 | 13 | 400 |
| P2 | 250 | 1000 | 50 | 1560 | 10 | 900 | 50 | 13 | 400 |
| P3 | 250 | 1000 | 50 | 1530 | 10 | 900 | 50 | 13 | 400 |
| P4 | 250 | 1150 | 50 | 1550 | 10 | 900 | 50 | 13 | 400 |
| P5 | 250 | 1150 | 50 | 1550 | 2 | 900 | 50 | 13 | 400 |
| P6 | 150 | 1000 | 50 | 1550 | 10 | 900 | 50 | 13 | 400 |
| P7 | 300 | 1000 | 50 | 1550 | 10 | 900 | 50 | 13 | 400 |
| P8 | 250 | 1000 | 100 | 1550 | 10 | 900 | 50 | 13 | 400 |
| P9 | 250 | 1000 | 50 | 1550 | 5 | 900 | 50 | 13 | 400 |
| P10 | 250 | 1000 | 50 | 1550 | 10 | 1050 | 50 | 10 | 400 |
| P11* | 250 | 1000 | 30 | 1550 | 10 | 900 | 50 | 13 | 400 |
| P12* | 250 | 1000 | 200 | 1550 | 10 | 900 | 50 | 13 | 400 |
| P13* | 250 | 1400 | 50 | 1550 | 10 | 900 | 50 | 13 | 400 |
| P14* | 250 | 1000 | 50 | 1550 | 0 | 900 | 50 | 13 | 400 |
| P15* | 250 | 1550 | | | 10 | 900 | 50 | 13 | 400 |
| P16* | 10 | 1500 | | | 120 | 25 | | | |
| In the table, "*" is outside the production method of this embodiment. | | | | | | | | | |

Reference examples

The calcined bodies of Synthesis examples 1 to 6 were sintered by normal sintering by the sintering pattern (P16) to obtain normal sintered bodies, and these sintered bodies were used as the sintered bodies of Reference examples 1 to 6. The results are indicated below.

[Table 3]

| | Calcined body | Total transmittance (%) |
|---|---|---|
| Reference example 1 | Synthesis example 1 | 42.1 |
| Reference example 2 | Synthesis example 2 | 45.1 |
| Reference example 3 | Synthesis example 3 | 26.0 |
| Reference example 4 | Synthesis example 4 | 48.4 |
| Reference example 5 | Synthesis example 5 | 42.0 |
| Reference example 6 | Synthesis example 6 | 32.0 |

It could be confirmed from Reference examples 1, 2 and 4 that increasing the yttrium content increased the total transmittance and improved the light transmitting property. In addition, it could be confirmed that a sintered body that contained a coloring element had a low total transmittance compared to a sintered body free of a coloring element.

Examples 1 to 6

The calcined bodies of Synthesis examples 1 to 6 were each sintered by the sintering pattern (P1) to obtain sintered bodies. The results are indicated below.

[Table 4]

|  | Calcined body | Total transmittance (%) | Transmittance ratio |
|---|---|---|---|
| Example 1 | Synthesis example 1 | 41.5 | 0.99 |
| Example 2 | Synthesis example 2 | 43.8 | 0.97 |
| Example 3 | Synthesis example 3 | 26.0 | 1.00 |
| Example 4 | Synthesis example 4 | 48.8 | 1.01 |
| Example 5 | Synthesis example 5 | 42.0 | 1.00 |
| Example 6 | Synthesis example 6 | 32.0 | 1.00 |

The sintered bodies of Examples containing 3 mol% or more and 5.2 mol% or less of yttrium had a transmittance ratio of 0.97 or more and 1.03 or less and had a light transmitting property about the same as those of normal sintered bodies. This could confirm that, according to the production method of these examples, a sintered body having a light transmitting property about the same as those of normal sintered bodies could be produced irrespective of the yttrium content, in particular, even when the yttrium content was 4.5 mol% or more.
Note that a sintered body of Example 4 having an yttrium amount of 5.2 mol% had a three-point bending strength of 836 MPa, and could be confirmed to have a strength required as a ceramic for a four-tooth or higher connected prosthesis specified in JIS T 6526.

Examples 7 to 10 and Comparative examples 1 to 4

The calcined body of Synthesis example 2 or 4 was sintered by the following sintering pattern to obtain a sintered body. The results are indicated in the table below.

[Table 5]

|  | Calcined body | Sintering pattern | Total transmittance (%) | Transmittance ratio |
|---|---|---|---|---|
| Example 7 | Synthesis example 2 | P1 | 43.8 | 0.97 |
| Example 8 | Synthesis example 2 | P8 | 44.8 | 0.99 |
| Comparative example 1 | Synthesis example 2 | P11 | 42.4 | 0.94 |
| Comparative example 2 | Synthesis example 2 | P12 | 45.1 | 1.00 |
| Example 9 | Synthesis example 4 | P1 | 48.8 | 1.01 |
| Example 10 | Synthesis example 4 | P8 | 47.4 | 0.98 |
| Comparative example 3 | Synthesis example 4 | P11 | 48.4 | 0.100 |
| Comparative example 4 | Synthesis example 4 | P12 | 44.3 | 0.92 |

It could be confirmed that, when HR2 was 200°C/minute and when the yttrium amount was 4 mol%, a sintered body having a light transmitting property comparable to that of a normal sintered body could be obtained whereas when the yttrium amount was 5.2 mol%, the light transmitting property was notably poor compared to a normal sintered body (Comparative examples 2 and 4). Conversely, it could be confirmed that, when HR2 was 30°C/minute and when the yttrium amount was 5.2 mol%, a sintered body having a light transmitting property comparable to that of a normal sintered body could be obtained whereas when the yttrium amount was 4 mol%, the light transmitting property was notably poor compared to a normal sintered body (Comparative examples 1 and 3). Furthermore, when a compositional substance having an yttrium amount of 4 mol% was used, the light transmitting property of the obtained sintered body increased with the increase in HR2 whereas when a compositional substance having an yttrium amount of 5.2 mol% was used, the light transmitting property of the obtained sintered body decreased with the increase in HR2; thus, it was confirmed that the relationship between HR2 and the light transmitting property differed depending on the yttrium amount.

Examples 11 to 16 and Comparative examples 5 and 6

The calcined body of Synthesis example 2 or 4 was sintered by the following sintering pattern to obtain a sintered body. The results are indicated in the table below.

[Table 6]

| | Calcined body | Sintering pattern | Total transmittance (%) | Transmittance ratio |
|---|---|---|---|---|
| Example 11 | Synthesis example 2 | P1 | 43.8 | 0.97 |
| Example 12 | Synthesis example 2 | P9 | 44.3 | 0.98 |
| Example 13 | Synthesis example 2 | P5 | 44.5 | 0.99 |
| Comparative example 5 | Synthesis example 2 | P14 | 41.7 | 0.92 |
| Example 14 | Synthesis example 4 | P1 | 48.8 | 1.01 |
| Example 15 | Synthesis example 4 | P9 | 47.1 | 0.97 |
| Example 16 | Synthesis example 4 | P5 | 46.4 | 0.96 |
| Comparative example 6 | Synthesis example 4 | P14 | 40.3 | 0.83 |

It could be confirmed that the light transmitting property decreased notably compared to a normal sintered body when retention at T2 was omitted irrespective of the yttrium amount (Comparative examples 5 and 6). Furthermore, when a compositional substance having an yttrium amount of 4 mol% was used, the light transmitting property of the obtained sintered body increased with the decrease in the retention time whereas when a compositional substance having an yttrium amount of 5.2 mol% was used, the light transmitting property of the obtained sintered body increased with the increase in the retention time; thus, it was confirmed that the influence of the retention time on the light transmitting property was different depending on the yttrium amount.

Examples 17 to 20 and Comparative examples 7 and 8

The calcined body of Synthesis example 2 or 4 was sintered by the following sintering pattern to obtain a sintered body. The results are indicated in the table below.

[Table 7]

| | Calcined body | Sintering pattern | Total transmittance (%) | Transmittance ratio |
|---|---|---|---|---|
| Example 17 | Synthesis example 2 | P1 | 43.8 | 0.97 |
| Example 18 | Synthesis example 2 | P4 | 44.6 | 0.99 |
| Comparative example 7 | Synthesis example 2 | P13 | 44.7 | 0.99 |
| Example 19 | Synthesis example 4 | P1 | 48.8 | 1.01 |
| Example 20 | Synthesis example 4 | P4 | 48.5 | 1.00 |
| Comparative example 8 | Synthesis example 4 | P13 | 43.9 | 0.91 |

It could be confirmed that, when T1 was 1400°C and when the yttrium content was 4 mol%, a sintered body having a light transmitting property comparable to that of a normal sintered body could be obtained whereas when the yttrium content was 5.2 mol%, the light transmitting property was notably poor compared to a normal sintered body.

Examples 21 to 26

The calcined body of Synthesis example 2 or 4 was sintered by the following sintering pattern to obtain a sintered body. The results are indicated in the table below.

[Table 8]

|  | Calcined body | Sintering pattern | Total transmittance (%) | Transmittance ratio |
|---|---|---|---|---|
| Example 21 | Synthesis example 2 | P3 | 44.7 | 0.99 |
| Example 22 | Synthesis example 2 | P1 | 43.8 | 0.97 |
| Example 23 | Synthesis example 2 | P2 | 44.6 | 0.99 |
| Example 24 | Synthesis example 4 | P3 | 47.3 | 0.98 |
| Example 25 | Synthesis example 4 | P1 | 48.8 | 1.01 |
| Example 26 | Synthesis example 4 | P2 | 48.5 | 1.00 |

It could be confirmed that when T2 was in the range of 1530°C or higher and 1560°C or lower, a light transmitting property comparable to that of a normal sintered body was obtained irrespective of the yttrium content.

Note that a sintered body of Example 24 having an yttrium amount of 5.2 mol% had a three-point bending strength of 955 MPa, and could be confirmed to have a strength required as a ceramic for a four-tooth or higher connected prosthesis specified in JIS T 6526:2018.

Examples 26 to 31

The calcined body of Synthesis example 2 or 4 was sintered by the following sintering pattern to obtain a sintered body. The results are indicated in the table below.

[Table 9]

|  | Calcined body | Sintering pattern | Total transmittance (%) | Transmittance ratio |
|---|---|---|---|---|
| Example 26 | Synthesis example 2 | P6 | 45.1 | 1.00 |
| Example 27 | Synthesis example 2 | P1 | 43.8 | 0.97 |
| Example 28 | Synthesis example 2 | P7 | 44.7 | 0.99 |
| Example 29 | Synthesis example 4 | P6 | 48.1 | 0.99 |
| Example 30 | Synthesis example 4 | P1 | 48.8 | 1.01 |
| Example 31 | Synthesis example 4 | P7 | 48.5 | 1.00 |

It could be confirmed that when HR was in the range of 150°C/minute or more and 300°C/minute or less, a sintered body having a light transmitting property comparable to that of a normal sintered body was obtained irrespective of the yttrium content.

The entire contents of the description, the claims, and the abstract of Japanese Patent Application No. 2022-106984 filed July 1st, 2022 are hereby incorporated by reference as the disclosure of the description of the present disclosure.

**Claims**

1. A method for producing a stabilizing element-containing zirconia sintered body, the method comprising: a first heating step of heating a zirconia compositional substance containing a stabilizing element from a heating start temperature to a first target temperature of 800°C or higher and lower than 1400°C at a heating rate of 150°C/minute or more; a second heating step of elevating the temperature from the first target temperature to a second target temperature of 1400°C or higher and lower than 1580°C at a heating rate of more than 30°C/minute and less than 200°C/minute; and a retaining step of retaining the second target temperature.

2. The production method according to claim 1, wherein a retention time in the retaining step is less than 20 minutes.

3. The production method according to claim 1 or 2, wherein a difference between the second target temperature and the first target temperature is 300°C or more and 600°C or less.

4. The production method according to any one of claims 1 to 3, wherein the heating rate up to the second target temperature relative to the heating rate up to the first target temperature is 0.15 or more and 1.0 or less.

5. The production method according to any one of claims 1 to 4, wherein the heating start temperature is a temperature ranging from room temperature to 500°C.

6. The production method according to any one of claims 1 to 5, further comprising a cooling step of decreasing the temperature from the second target temperature to a cooling temperature of 800°C or higher and 1200°C or lower and discharging a sintered body from a sintering furnace.

7. The production method according to claim 6, wherein the discharged sintered body is cooled in an air atmosphere by at least one of natural cooling and blowing of a cooling gas.

8. The production method according to claim 7, wherein the cooling gas is at least one selected from the group consisting of air, argon, nitrogen and helium.

9. The production method according to any one of claims 1 to 8, wherein a sintering furnace equipped with a heater containing at least one selected from the group consisting of molybdenum disilicide, silicon carbide, lanthanum chromite and carbon is used in sintering.

10. The production method according to any one of claims 1 to 9, wherein the compositional substance is a green body or calcined body of zirconia.

11. The production method according to any one of claims 1 to 10, wherein the stabilizing element is at least one selected from the group consisting of yttrium, calcium, cerium, magnesium, praseodymium, ytterbium, erbium and terbium.

12. The production method according to any one of claims 1 to 11, wherein a content of the stabilizing element is 2.5 mol% or more and 8 mol% or less.

13. The production method according to any one of claims 1 to 12, wherein the compositional substance has a T + C phase ratio of 65% or more.

14. The production method according to any one of claims 1 to 13, wherein the compositional substance contains at least one coloring element selected from transition metal elements other than zirconium and hafnium and lanthanoid rare earth elements.

## INTERNATIONAL SEARCH REPORT

| | International application No. |
|---|---|
| | **PCT/JP2023/024507** |

### A. CLASSIFICATION OF SUBJECT MATTER

*C04B 35/486*(2006.01)i; *A61K 6/818*(2020.01)i; *A61K 6/822*(2020.01)i
FI:   C04B35/486; A61K6/818; A61K6/822

According to International Patent Classification (IPC) or to both national classification and IPC

### B. FIELDS SEARCHED

Minimum documentation searched (classification system followed by classification symbols)

C04B35/486; A61K6/818; A61K6/822

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2021/020582 A1 (KURARAY NORITAKE DENTAL INC.) 04 February 2021 (2021-02-04)<br>    paragraphs [0003], [0006], [0011], [0022], [0033]-[0035], [0040], [0056], example 3, table 3 | 1-12, 14 |
| A | paragraph [0029] | 13 |
| A | JP 2009-207901 A (IVOCLAR VIVADENT AG) 17 September 2009 (2009-09-17)<br>    entire text, all drawings | 1-14 |
| A | JP 2022-068261 A (KURARAY NORITAKE DENTAL INC.) 09 May 2022 (2022-05-09)<br>    entire text, all drawings | 1-14 |
| A | WO 2020/178712 A1 (3M INNOVATIVE PROPERTIES COMPANY) 10 September 2020 (2020-09-10)<br>    entire text, all drawings | 1-14 |
| A | JP 1-148748 A (MITSUBISHI MINING & CEMENT CO LTD) 12 June 1989 (1989-06-12)<br>    entire text, all drawings | 1-14 |

☐ Further documents are listed in the continuation of Box C.     ☑ See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **05 September 2023** | **19 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/024507**

| Patent document cited in search report | | | Publication date (day/month/year) | Patent family member(s) | | | Publication date (day/month/year) |
|---|---|---|---|---|---|---|---|
| WO | 2021/020582 | A1 | 04 February 2021 | US 2022/0267215 A1 paragraphs [0003], [0006], [0020], [0033], [0040], [0042]-[0044], [0049], [0063], example 3, table 3 EP 4008699 A1 CN 114144389 A KR 10-2022-0042345 A | | | |
| JP | 2009-207901 | A | 17 September 2009 | US 2009/0226855 A1 entire text, all drawings EP 2098188 A1 | | | |
| JP | 2022-068261 | A | 09 May 2022 | WO 2022/065452 A1 entire text, all drawings | | | |
| WO | 2020/178712 | A1 | 10 September 2020 | CN 113508100 A entire text, all drawings JP 2022-525738 A | | | |
| JP | 1-148748 | A | 12 June 1989 | (Family: none) | | | |

Form PCT/ISA/210 (patent family annex) (January 2015)

**REFERENCES CITED IN THE DESCRIPTION**

**Patent documents cited in the description**

- WO 2021048674 A
- WO 2019166938 A

- JP 2022106984 A